# EUROPEAN PATENT SPECIFICATION

(11) **EP 1 165 464 B1**
(45) Date of publication and mention of the grant of the patent: **20.08.2003**
(21) Application number: 00912791.1
(22) Date of filing: 24.03.2000
(51) Int. Cl.: C07B 39/00, C07C 17/10, C07C 67/307

(54) **PREPARATION OF SELECTIVELY FLUORINATED ORGANIC COMPOUNDS**
VERFAHREN ZUR SELEKTIVEN FLUORIERUNG VON ORGANISCHEN VERBINDUNGEN
PREPARATION DE COMPOSES ORGANIQUES FLUORES DE FA ON SELECTIVE

(30) Priority: 30.03.1999 GB 9907214
(43) Date of publication of application: 02.01.2002
(73) Proprietor: F2 Chemicals Limited, Preston, Lancashire PR4 0RZ (GB)
(72) Inventor: Chambers, Richard Dickinson, Durham DH1 3LE (GB); Sandford, Graham, Durham DH1 3LE (GB); Parsons, Mandy, Durham DH1 3LE (GB)
(74) Representative: Jones, Keith William
(86) International application number: GB0001121
(87) International publication number: WO00058241

(56) References cited:
- US-A- 5 086 178

## Description

The present invention relates to the selective fluorination or organic compounds.

Most reported reactions with electrophilic fluorinating reagents involve transformation of carbon-hydrogen bonds to carbon-fluorine bonds where the hydrogen atom is attached to either a carbon atom that is sp² hybridised (alkenes, aromatics, carbonyl derivatives, etc) or to a carbon atom that bears a negative charge (carbanions, etc) or which must form, for example, an enol before fluorination can occur as with β-dicarbonyl compounds.

Selective transformation of carbon-hydrogen bonds to carbon-fluorine bonds, in which the hydrogen atom is attached to a fully saturated unactivated sp³ hybridised carbon atom, in a simple, one-step process would be particularly advantageous but fluorination of saturated systems is very difficult. Conversion of carbon-hydrogen bonds to carbon-fluorine bonds at low temperature in saturated systems such as decalin and various steroid derivatives using elemental fluorine has been reported in *Tetrahedron Lett.,* 1984, **25***,* 1947. However, fluorine is a corrosive, toxic gas and requires the use of specialist equipment.

In US-A-5 086 178 are disclosed compounds having an N-F bond as electrophilic fluorinating agents. However, there is no disclosure that such compounds could be used to selectively fluorinate an organic compound at an unactivated sp³ hybridised carbon atom.

According to the present invention there is provided a method of selectively fluorinating an organic compound at an unactivated saturated sp³ hybridised carbon atom, the method comprising reacting the compound with a fluorinating agent containing an N-F bond.

The present invention enables direct fluorination of a saturated sp³ hybridised carbon atom to be accomplished efficiently without the disadvantages associated with using fluorine gas described above.

The method typically comprises substituting a hydrogen atom on the saturated sp³ hybridised carbon atom by a fluorine atom.

The saturated sp³ hybridised carbon atom may be bonded to three other carbon atoms and a hydrogen atom, i.e. a tertiary carbon atom.

The saturated sp³ hybridised carbon atom may be bonded to two other carbon atoms and two hydrogen atoms, i.e. a secondary carbon atom.

The saturated carbon atom may form part of a cyclic structure.

Where the saturated carbon atom is a tertiary carbon atom it may form part of a cyclic structure such as decalin or adamantane. Where the saturated carbon atom is a secondary carbon atom it may form part of a cyclic structure.

Advantageously the saturated carbon atom does not need to be bonded to groups such as carbonyl groups as in the fluorination of e.g. β-dicarbonyl compounds.

The term unactivated saturated sp³ carbon atom used herein means a saturated sp³ hybridised carbon atom not directly bonded to any functional group. That is to say, any carbon atom which is part of a functional group (e.g. carbonyl C=O) or is bearing a functional group (e.g. C-OH) must be at least two atoms distant from the carbon which is bonded to the hydrogen atom that is replaced by fluorine. In the prior art, functional groups such as for example carbonyl were directly bonded, i.e. less than two carbons distant, to the carbon atom which is bonded to the hydrogen atom that is replaced by fluorine. Thus, in the prior art, the functional group would activate the carbon-hydrogen bond on the saturated carbon atom.

The fluorinating agent may be any clectrophilic fluorinating agent containing an N-F bond.

The fluorinating agent may, in particular, comprise one of the following : an N-fluoropiperidine, an N-fluoro pyridone, an N-fluorosulphonamide, an N-fluoropyridinium salt, an N-fluoroimide or an N-fluorodiazabicycloalkane or similar reagents.

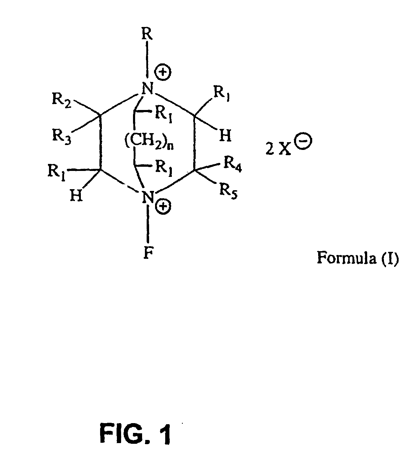

Preferably, the fluorinating agent is an N-fluorinated diazoniabicycloalkane, i.e. one of the class of reagents known commercially as Selectfluor (Trade mark)reagents. A Selectfluor (Trade mark) reagent is shown schematically in Figure 1.

More preferably the fluorinating agent is an N-fluorinated 1,4-diazoniabicyclo-[2.2.2]octane derivative.

Most preferably the fluorinating agent is a 1-alkyl-4-fluoro-1,4-diazoniabicyclo[2.2.2]octane salt, particularly a 1-chloromethyl-4-fluoro-1,4-diazabicyclo[2.2.2]octane salt. Preferably the salt is a bis(tetrafluoroborate)salt.

Selectfluor (Trade mark) reagent have been described previously in detail, for example, in US patent no. 5,442,084. Known preferred substituents, counter ions and other features of Selectfluor (Trade mark) reagents are incorporated herein by reference. In particular, there is incorporated herein by reference those features described in US patent no. 5,442,084 at column 3 lines 11 to 25 and at column 5 line 36 to column 7 line 10.

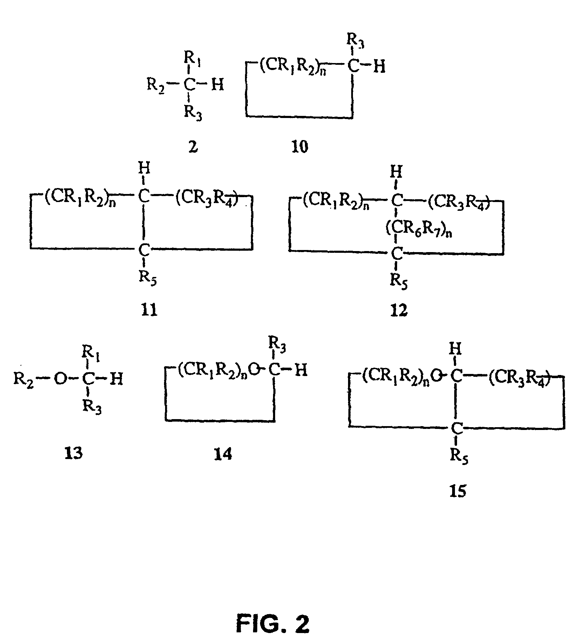

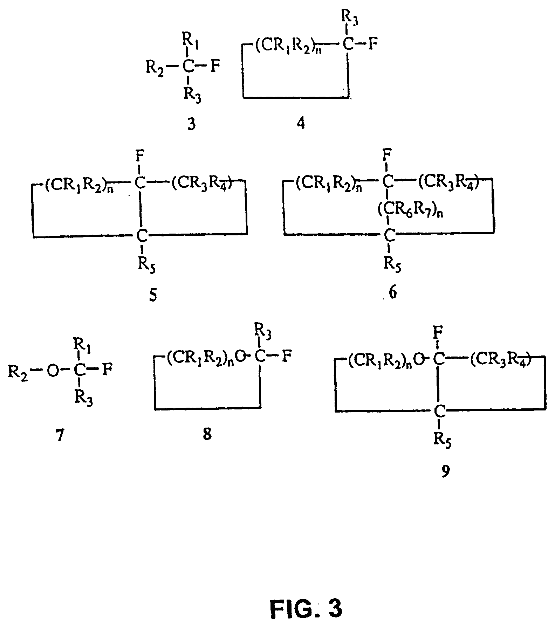

The present invention may be used to convert a wide range of organic substrates such as **2,10,11,12,13,14** and **15** shown in Figure 2 to the corresponding fluorinated analogues **3,4,5,6,7,8** and **9** shown in Figure 3.

In structures **2, 10, 11, 12, 13, 14, 15** and **3, 4, 5, 6, 7, 8, 9,** the groups R₁, R₂, R₃, R₄, R₅, R₆ and R₇ may be selected from hydrogen, alkyl, substituted alkyl, cycloalkyl, substituted cycloalkyl, aryl, substituted aryl, etc. Where any of the groups R₁, R₂, R₃, R₄, R₅, R₆ and R₇ is an alkyl, cycloalkyl or aryl substituent, the said group may include one or more optional substituents or hetero-atoms.

The structures represented by formulae **4, 5, 6, 8, 9, 10, 11, 12, 14** and **15** are cyclic and n may be an integer in the inclusive range from 1 to 8.

Preferably, the fluorination is conducted in a solvent. Preferably the solvent comprises a largely inert substance such as acetonitrile, nitromethane, formic acid, dichloromethane, etc., though not limited to these solvents. Solvents comprising tetrahydrofuran, dimethylformamide, hexane, or diethyl ether may also be used.

The reaction of the process may be carried out at a temperature in the range of -60°C to +150°C although a temperature of from 0°C to 100°C is preferred.

The ratio of fluorinating reagent to organic compound of formula **2, 10, 11, 12, 13, 14** or **15** may be varied within wide limits although it is preferred that the molar ratio is in the range 0.5 to 10:1, especially 1 to 3:1.

When the fluorination reaction of the process is complete, the selectively fluorinated organic compounds, e.g. **3, 4, 5, 6, 7, 8** and **9** may be isolated by addition of water to the cooled reaction followed by extraction of the selectively fluorinated organic compound into a suitable solvent followed by, for example, purification by distillation or column chromatography.

Embodiments of the present invention will now be described, by way of example only, with reference to the following examples.

### Example 1: Preparation of 1-Fluoroadamantane

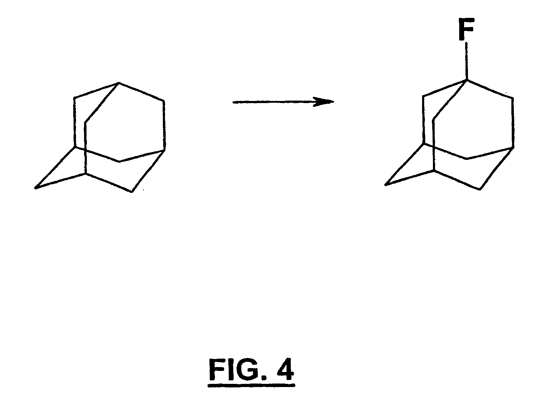

1-fluoroadamantane shown in scheme in Figure 4 was prepared as follows. A solution containing selectfluor™ (4.69g, 13 mmol) and adamantanc (1.00g, 7 mmol) in dry acetonitrile (100 cm³) was stirred at reflux temperature. After 3 h the reaction mixture was poured into water, neutralised (NaHCO₃) and extracted three times using DCM. The combined dried (MgSO₄) organic extracts were evaporated to give a brown solid. Column chromatography on silica gel using 6:1 cyclohexane-DCM was used to isolate 1-fluoroadamantane (0.23g, 30%, 73% conv.) as a white solid from this crude product; mp 256-258°C (Lit. 257-259°C); δ_{F} (376 Mhz) 128.8 (s); δ_{H} (400 Mhz) 1.69 (2H, m, 4-H), 1.95 (2 H, m, 2-H), 2.16 (1 H, s, 3-H); δ_{C} (101 Mhz) 31.5 (d, ³J_{CF} 9.6, C-3), 35.8 (d, ⁴J_{CF} 1.9, C-4), 42.7 (d, ²J_{CF} 17.1, C-2), 92.6 (d, ¹J_{CF} 183.2, C-1); *m*/*z* (EI⁺) 154 (M⁺, 75%).

### Example 2: Preparation of Fluorocyclohexane

A solution containing Selectfluor™ (13.91 g, 39 mmol), cyclohexane (3.00 g, 36 mmol) and dry acetonitrile (130 cm³) was stirred and heated (65°C). After 26 h the reaction mixture was poured into water, neutralised (NaHCO₃) and extracted with dichloromethane (3 x 50 ml). The combined, dried (MgSO₄) organic extracts were evaporated to give a colourless product (8.00 g) which contained fluorocyclohexane (0.77 g, 21%, 100% conv.). Purification of the crude product by preparative scale GC gave an analytically pure sample of fluorocyclohexane as a colourless oil; bp 101-103°C (Lit.,[Ashton, 1971 #109] 62°C/180mmHg) (Found: C, 69.5; H, 10.7, M⁺, 102.0845. C₆H₁₁F requires C, 70.53; H, 10.9%, M⁺, 102.0845); δ_{F} (376 MHz) -171.47 (br s); δ_{H} (400 MHz) 1.30-1.75 (10 H, m, 2-H, 3-H and 4-H), 4.57 (1 H, dm, ²J_{HF} 48.8, J_{HH} 3.6, 1-H); δ_{C}(100 MHz) 22.8 (d, ³J_{CF} 7.7, C-3), 25.2 (d, ⁴J_{CF} 1.5, C-4), 32.3 (d, ²J_{CF} 18.7, C-2), 91.5 (d, ¹J_{CF} 169.8, C-1); *m*/*z* (EI⁺) 102 (M⁺, 8%), 82 (40, M⁺-HF).

### Example 3: Fluorination of trans-decalin

A solution containing Selectfluor™ (24.85 g, 70 mmol), *trans*-decalin (5.38 g, 70 mmol) and dry acetonitrilc (250 cm³) was stirred at reflux temperature. After 4.5 h the reaction mixture was worked up as above to give a brown liquid (4.37 g) which contained *trans*-fluorodecalin (2.21 g, 25%, 81% conv.) (31 area %), *trans*-decalin (65 area %) and, trace amounts of other products. Purification of the crude product by preparative scale GC gave an analytically pure sample of *trans*-fluorodecalin as an isomeric mixture of 2-fluoro_{(eq)}decalin (isomer 3), 1-fluoro_{(eq)}decalin (isomer 2), 2-fluoro₍ₐₓ₎decalin (isomer 4) and 1-fluoro₍ₐₓ₎decalin (isomer 1) in the ratio of 1.36 : 1.14 : 2.24 : 1:00 respectively and, as a colourless oil (Found: C, 77.0; H, 11.1. C₁₀H₁₇F requires C, 76.9; H, 11.0%); δ_{F} (376 MHz) -167.97 (dd, ²J_{HF} 49.4, ³J_{HF} 4.5, isomer 3), -177.34 (d, ²J_{HF} 49.3, isomer 2), -183.07 (tq, ²J_{HF} 47.8, ³J_{HF} 10.2, isomer 4), -196.61 (mq, ²J_{HF} 49.6, isomer 1); δ_{H} (400 MHz) 0.60-2.10 (113.97 H, m, CH₂ and CH), 4.08 (1.16 H, dddd, ²J_{HF} 49.8, ³J_{HF} 10.8, ³J_{HF} 9.9, ³J_{HF} 4.8, CHF-isomer 2), 4.47 (1.47 H, dtt, ²J_{HF} 49.2, ³J_{HF} 10.8, ³J_{HF} 4.8, CHF-isomer 3), 4.54 (1.00 H, ddt, ²J_{HF} 49.2, ³J_{HF} 3.2, ³J_{HF} 2.0, CHF-isomer 1), 4.87 (2.40 H, d sept, ²J_{HF} 48.4, ³J_{HF} 2.0, CHF-isomer 4); δ_{C} (101 MHz) 20.5 (s, CH₂-isomer 1), 23.4 (s, CH₂-isomer 1 or 2), 23.5 (s, CH₂-isomer 1 or 2), 25.9 (s, CH₂-isomer 2), 26.4 (d, ⁴J_{CF} 0.7, CH₂-isomer 3), 26.5 (s, CH₂), 26.6 (s, CH₂), 26.7 (s, CH₂), 26.8 (s, CH₂), 26.9 (s, CH₂), 27.0 (s, CH₂), 27.9 (s, CH₂-isomer 4), 28.9 (d, ³J_{CF} 2.7, CH₂-isomer 2), 29.3 (d, ³J_{CF} 2.3, CH₂-isomer 1), 31.1 (d, ²J_{CF} 21.3, CH₂-isomer 4), 31.4 (d, 11.5, CH₂-isomer 3), 31.7 (d, ²J_{CF} 21.7, CH₂-isomer 1), 32.8 (s, CH₂-isomer 2), 33.1 (d, ²J_{CF} 17.6, CH₂-isomer 2), 33.2 (d, 12.7, CH₂-isomer 2), 33.5 (s, CH₂-isomer 1 or 4), 33.5 (s, CH₂-isomer 1 or 4), 33.9 (s, CH₂-isomer 4), 33.9 (d, ³J_{CF} 2.3, CH₂-isomer 4), 34.4 (s, CH2), 36.4 (s, CHR₃-isomer 1), 36.9 (s, CHR₃-isomer 4), 38.5 (d, ²J_{CF} 21.4, CH₂-isomer 4), 40.3 (d, ²J_{CF} 16.4, CH₂-isomer 2), 40.8 (d, 10.7, CHR₃-isomer 2), 40.9 (d, 9.2, CHR₃-isomer 3), 42.3 (d, ⁴J_{CF} 1.9, CHR₃-isomer 2), 42.7 (d, CHR₃-isomer 4), 46.6 (d, ²J_{CF} 19.3, CHR₃-isomer 1), 48.8 (d, ²J_{CF} 16.3, CHR₃-isomer 3), 90.6 (d, ¹J_{CF} 165.0, CHF-isomer 4), 92.6 (d, ¹J_{CF} 170.4, CHF-isomer 3), 93.2 (d, ¹J_{CF} 168.4, CHF-isomer 1), 96.7 (d, ¹J_{CF} 171.4, CHF-isomer 2); *m*/*z* (EI⁺) 156 (M⁺, 82%), 136 (76, M⁺-HF), all isomers gave a similar result.

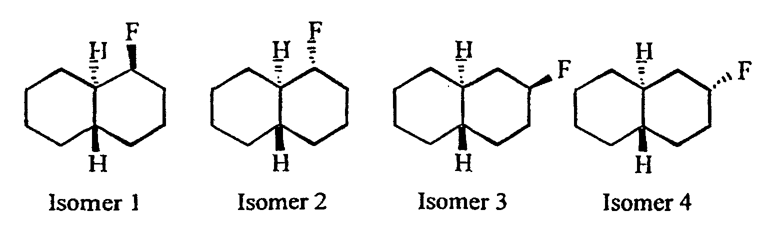

### Example 4: Fluorination of cis-decalin

A solution containing Selectfluor™ (13.85 g, 39 mmol), *cis*-decalin (3.00 g, 22 mmol) and dry acetonitrile (120 cm³) was stirred at reflux temperature. After 1.5 h the reaction mixture was worked up as before to give a yellow liquid (3.20 g) which contained *cis*-fluorodecalin (0.77 g, 30%, 75% conv.) (39 area %), *cis*-decalin (43 area %) and, trace amounts of other products. Purification of the crude product by preparative scale GC gave an analytically pure sample of *cis*-fluorodecalin as an isomeric mixture of 1-fluoro₍ₐₓ₎decalin (isomer 1), 1-fluoro_{(eq)}decalin (isomer 2), 2-fluoro_{(eq)}decalin (isomer 3) and 2-fluoro₍ₐₓ₎decalin (isomer 4) in the ratio of 1.60 : 1.40 : 1.30 : 1:00 not necessarily respectively and, as a colourless oil (Found: C, 77.0; H, 11.1. C₁₀H₁₇F requires C, 76.9; H, 11.0%); δ_{F} (376 MHz, -57°C)-166.55 (d, ²J_{HF} 47.8), -173.43 (d, ²J_{HF} 49.3), -179.08 (d, ²J_{HF} 49.6), -183.92 (q, ²J_{HF} 37.9); δ_{H} (400 MHz, -57°C) 1.07-2.06 (16 H, m, CH and CH₂), 4.45-4.84 (1 H, m, CHF); δ_{C} (101 MHz, -57°C) 18.0-42.0 (many s and m, CH and CH2), 90.5 (d, ¹J_{CF} 171.7, CHF), 90.8 (d, ¹J_{CF} 165.5, CHF), 94.1 (d, ¹J_{CF} 170.5, CHF), 94.3 (d, ¹J_{CF} 166.0, CHF); *m*/*z* (EI⁺) 156 (M⁺, 22%), 136 (86, M⁺-HF), all isomers gave a similar result.

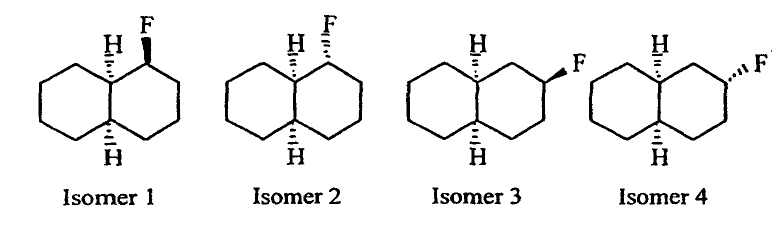

### Example 5: Fluorination of decane

A solution containing Selectfluor™ (21.42 g, 61 mmol), decane (7.81 g, 55 mmol) and dry acetonitrile (210 cm³) was stirred and heated (82°C). After 18 h the reaction mixture was worked up as above to give a orange liquid (10.77 g) which contained fluorodecane (4.28 g, 58%, 84% conv.) (50 area %), decane (16 area %) and, small amounts of other unidentified products. Purification by preparative scale GC gave an isomeric mixture of 2-, 3-, 4- and 5-fluorodecane in the ratio of 2.39 : 1.27 : 1.09 : 1.00 not necassarily respectively and, as a colourless liquid (Found: M⁺-HF, 140.1565. C₁₀H₂₁F requires M⁺-HF, 140.1565); δ_{F} (376 MHz) -172.46 (m, J_{HF} 19.2), -180.38 (m, J_{HF} 17.3), -180.71 (m, J_{HF} 17.2), -181.62 (m, J_{HF} 18.8); δ_{H} (400 MHz) 0.85-0.98 (5 H, m, CH₂ and/or CH₃), 1.24-1.70 (15 H, m, CH₂ and/or CH₃), 4.30-4.74 (1.00 H, m, CHF); δ_{C}(100 MHz) 9.4 (s, CH₃), 9.42 (s, CH₃), 13.9 (s, CH₃), 14.0 (s, CH₃), 14.1 (s, CH₃), 18.4 (d, ³J_{CF} 4.6, CH₂), 21.0 (d, ²J_{CF} 22.9, CH₃), 22.6 (s, CH₂), 22.7 (s, CH₂), 24.8 (d, ³J_{CF} 4.6, CH₂), 25.1 (d, ³J_{CF} 5.0, CH₂), 27.3 (d, ³J_{CF} 4.6, CH₂), 28.1 (d, ²J_{CF} 21.3, CH₂), 29.2 (d, ³J_{CF} 2.6, CH₂), 29.2 (s, CH₂), 29.5 (d, ⁴J_{CF} 1.2, CH₂), 29.7 (s, CH₂), 31.7 (d, ³J_{CF} 4.2, CH₂), 31.8 (d, ³J_{CF} 4.1, CH₂), 31.9 (s, CH₂), 34.7 (d, ²J_{CF} 20.5, CH₂), 34.9 (d, ²J_{CF} 20.9, CH₂), 35.1 (d, ²J_{CF} 21.0, CH₂), 35.2 (d, ²J_{CF} 20.5, CH₂), 36.9 (d, ²J_{CF} 20.6, CH₂), 37.3 (d, ²J_{CF} 21.0, CH₂), 91.1 (d, ¹J_{CF} 164.1, CHF), 94.4 (d, ¹J_{CF} 166.4, CHF), 94.6 (d, ¹J_{CF} 166.4, CHF), 95.8 (d, ¹J_{CF} 166.1, CHF); *m*/*z* (EI⁺) 140 (M⁺-HF, 1%), 111 (9), 97 (22), all isomers gave a similar result.

### Example 6: Fluorination of Methyl valerate

A mixture containing Selectfluor™ (13.42 g, 38 mmol), methyl valerate (4.00 g, 34 mmol) and dry acetonitrile (130 cm³) was stirred and heated (82°C). After 16 h the reaction mixture was worked up as above to give a colourless product (10.05 g) which contained methyl valerate (55 area %); methyl 4-fluorovalerate and methyl 3-fluorovalerate (0.94 g, 49%, 42% conv.) (32 area %) and, small amounts of other unidentified products. Purification by preparative scale GC gave a sample of methyl 4-fluorovalerate and methyl 3-fluorovalerate as an isomeric mixture in the ratio of 1.2 : 1.0 respectively and, as a colourless oil (Found: M⁺+NH₄, 152.1092. C₆H₁₁FO₂ requires: M⁺+NH₄, 152.1087); methyl 4-fluorovalerate δ_{F} (376 MHz) -173.97 (m); δ_{H} (400 MHz) 1.31 (3 H, d, ³J_{HH} 6.4, 5-H), 1.78-1.90 (2 H, m, 3-H), 2.40 (2 H, m, 2-H), 3.62 (3 H, s, OCH₃), 4.62 (1 H, dm, ²J_{HF} 49.2, 4-H); δ_{C}(100 MHz) 20.8 (d, ²J_{CF} 22.6, C-5), 29.6 (d, ³J_{CF} 4.5, C-2), 31.9 (d, ²J_{CF} 21.4, C-3), 51.6 (s, OCH₃), 89.9 (d, ¹J_{CF} 166.2, C-4), 173.5 (s, C-1); *m*/*z* (EI⁺) 114 (M⁺-HF, 8%), 103 (27), 83 (20), 74 (23); methyl 3-fluorovalerate δ_{F} (376 MHz) -179.81 (m); δ_{H} (400 MHz) 0.98 (3 H, t, ³J_{HH} 7.2, 5-H), 1.65 (2 H, m, 4-H), 2.63 (2 H, m, 2-H), 3.65 (3 H, s, OCH₃), 4.81(1 H, dm, ²J_{HF} 48.4, 4-H); δ_{C}(100 MHz) 9.0 (s, C-5), 27.8 (d, ²J_{CF} 21.0, C-4), 39.8 (d, ²J_{CF} 24.1, C-2), 51.8 (s, OCH₃), 91.4 (d, ¹J_{CF} 170.9, C-3), 174.3 (s, C-1); *m*/*z* (EI⁺) 114 (M⁺-HF, 8%), 103 (27), 83 (20), 74 (23).

### Example 7: Fluorination of Methyl enanthate

A mixture containing Selectfluor™ (13.66 g, 31 mmol), methyl enanthate (4.00 g, 28 mmol) and dry acetonitrile (140 cm³) was stirred and heated (82°C). After 16 h the reaction mixture was worked up as above to give a yellow product (4.68 g) which contained methyl enanthate (49 area %), methyl 3-, 4-, 5- and 6-fluoroenanthate (1.24 g, 48%, 57% conv.) (42 area %) and, small amounts of other unidentified products. Purification of the crude product by preparative scale GC gave an analytically pure sample of methyl 6-fluoroenanthate (A), methyl 5-fluoroenanthate (B), methyl 4-fluoroenanthate (C) and methyl 3-fluoroenanthate (D) in the ratio of 3.7 : 1.3 : 1.3 : 1:0 respectively and, as a colourless liquid (Found: C, 59.1; H, 9.3. C₈H₁₅FO₂ requires C, 59.2; H, 9.3%); δ_{F} (376 MHz) -173.14 (m, A), -180.08 (m, C), -182.8 (m, B), -183.62 (m, D); δ_{H} (400 MHz) 0.80-1.00 (1 H, m, CH₂ and/or CH₃), 1.27-1.65 (7 H, m, CH₂ and/or CH₃), 2.30-2.38 (3 H, m, CH₂ and/or CH₃), 3.67-6.68 (3 H, m, OCH₃), 4.25-5.02 (1 H, m, CHF); δ_{C}(100 MHz) 9.6 (d, J_{CF} 4.9, CH₃-B, C or D), 14.1 (s, CH₃-B, C or D), 14.2 (s, CH₃-B, C or D), 18.5 (s, CH₂), 18.6 (s, CH₂), 20.9 (d, J_{CF} 4.1, CH₂), 21.2 (d, ²J_{CF} 22.9, CH₃-A), 24.9 (d, J_{CF} 5.0, CH₂), 27.2 (d, J_{CF} 4.2, CH₂), 28.5 (d, ²J_{CF} 22.6, CH₂CHF), 29.9 (d, J_{CF} 4.2, CH₂), 30.5 (d, ²J_{CF} 21.4, CH₂CHF), 33.9 (s, CH₂), 34.2 (d, ²J_{CF} 21.0, CH₂CHF), 34.8 (d, ²J_{CF} 20.2, CH₂CHF), 36.8 (d, ²J_{CF} 20.6, CH₂CHF-A), 37.4 (d, ²J_{CF} 20.6, CH₂CHF), 40.5 (d, ²J_{CF} 24.0, CH₂CHF), 51.7 (s, OCH₃), 51.8 (s, OCH₃), 51.8 (s, OCH₃), 51.9 (s, OCH₃), 90.6 (d, ¹J_{CF} 169.4, CHF-C), 90.7 (d, ¹J_{CF} 164.5, CHF-A), 92.4 (d, ¹J_{CF} 167.9, CHF-D), 95.4 (d, ¹J_{CF} 167.5, CHF-B), 173.9 (s, CO-D), 174.1 (s, CO-B), 174.3 (s, CO-A), 174.6 (s, CO-C); *m*/*z* (CI⁺, NH₃) 180 (M⁺ + 18, 100%), all isomers gave a similar result.

### Example 8: Fluorination of 1-Chlorohexane

A mixture containing Selectfluor™ (25.96 g, 73 mmol), 1-chlorohexane (8.00 g, 67 mmol) and dry acetonitrile (260 cm³) was stirred and heated (82°C). After 16 h the reaction mixture was worked up as above to give a dark yellow product (8.24 g) which contained 1-chlorohexane (59 area %), 1-chloro-2-, 3-, 4- and 5-fluorohexane (2.75 g, 56%, 53% conv.) (37 area %) in the ratio of 1.0 : 2.7 : 5.2 : 10.1 respectively; δ_{F} (188 MHz) -171.50 (m, 1-chloro-5-fluorohexane), -180.99 (m, 1-chloro-4-fluorohexane), -181.80 (m, 1-chloro-2-fluorohexane), -183.80 (m, 1-chloro-3-fluorohexane) and, small amounts of other unidentified products. Purification by preparative scale GC gave an analytically pure sample of 1-chloro-5-fluorohexane and 1-chloro-4-fluorohexane as an isomeric mixture and, as a colourless oil (Found: C, 51.8; H, 8.7. C₆H₁₂CIF requires C, 52.0; H, 8.7%); δ_{F} (376 MHz)-173.29 (m, 1-chloro-5-fluorohexane), -182.64 (m, 1-chloro-4-fluorohexane); δ_{H} (400 MHz) 0.90-1.00 (4.7 H, m, CH₂ and/or CH₃), 1.29-2.10 (37.6 H, m, CH₂ and/or CH₃), 3.55 (11.4 H, m, CH₂Cl), 4.42 (1 H, dm, ²J_{HF} 49.5, CHF-1-chloro-4-fluorohexane), 4.68 (3.7 H, dm, ²J_{HF} 48.5, CHF-1-chloro-5-fluorohexane); 1-chloro-5-fluorohexane δ_{C}(100 MHz) 21.0 (d, ²J_{CF} 20.9, C-6), 22.5 (d, ³J_{CF} 5.0, C-3), 32.3 (s, C-2), 36.1 (d, ²J_{CF} 21.0, C-4), 44.8 (s, C-1), 90.7 (d, ¹J_{CF} 164.4, C-5); *m*/*z* (EI⁺) no indicative peaks observed; 1-chloro-4-fluorohexane δ_{C}(100 MHz) 9.3 (d, ³J_{CF} 6.4, C-6), 28.1 (d, ²J_{CF} 21.0, C-3 or C-5), 28.3 (d, ³J_{CF} 3.8, C-2), 32.0 (d, ²J_{CF} 21.3, C-3 or C-5), 44.9 (s, C-1), 94.9 (d, ¹J_{CF} 168.3, C-4); *m*/*z* (EI⁺) no indicative peaks observed.

### Example 9: Fluorination of 1-Bromohexane

A mixture containing Selectfluor™ (11.80 g, 33 mmol), 1-bromohexane (5.00 g, 30 mmol) and dry acetonitrile (120 cm³) was stirred and heated (82°C). After 16 h the reaction mixture was worked up as above to give a dark yellow product (7.93 g) which contained 1-bromohexane (69 area %), 1-bromo-3-, 4- and 5-fluorohexane (1.10 g, 75%, 30% conv.) (21 area %) in the ratio of 1.0 : 1.8 : 4.8 respectively; δ_{F} (188 MHz) -172.34 (m, 1-bromo-5-fluorohexane), -181.74 (m, 1-bromo-4-fluorohexane), -185.00 (m, 1-bromo-3-fluorohexane) and, a large number of other unidentified products (10 area %). Purification by preparative scale GC gave an analytically pure sample of 1-bromo-5-fluorohexane and 1-bromo-4-fluorohexane as an isomeric mixture and, as a colourless oil (Found: C, 39.6; H, 6.7. C₆H₁₂BrF requires: C, 39.4; H, 6.6%); δ_{F} (376 MHz) -171.83 (m, 1-bromo-5-fluorohexane), -181.18 (m, 1-bromo-4-fluorohexane); δ_{H} (400 MHz) 0.85-1.00 (3.5 H, m, CH₂ and/or CH₃), 1.29-2.05 (28 H, m, CH₂ and/or CH₃), 3.42 (7 H, m, CH₂Br), 4.43 (2.5 H, dm, ²J_{HF} 49.2, CHF-1-bromo-5-fluorohexane), 4.66 (1 H, dm, ²J_{HF} 48.8, CHF-1-bromo-4-fluorohexane); 1 -bromo-5-fluorohexane δ_{C}(100 MHz) 21.0 (d, ²J_{CF} 22.8, C-6), 23.8 (d, ³J_{CF} 4.6, C-3), 32.5 (s, C-2), 33.5 (s, C-1), 35.9 (d, ²J_{CF} 20.6, C-4), 90.7 (d, ¹J_{CF} 164.9, C-5); *m*/*z* (EI⁺) no indicative peaks observed; 1-bromo-4-fluorohexane δ_{C}(100 MHz) 9.3 (d, ³J_{CF} 5.7, C-6), 28.1 (d, ²J_{CF} 21.0, C-3 or C-5), 28.4 (d, ³J_{CF} 3.8, C-2), 33.2 (d, ²J_{CF} 20.9, C-3 or C-5), 33.6 (s, C-1), 94.8 (d, ¹J_{CF} 168.3, C-4); *m*/*z* (EI⁺) no indicative peaks observed.

## Claims

1. A method of selectively fluorinating an organic compound at an unactivated saturated sp³ hybridised carbon atom, the method comprising reacting the compound with a fluorinating agent containing an N-F bond.

2. A method as in claim 1 wherein a hydrogen atom at the saturated carbon atom is substituted by a fluorine atom.

3. A method as in claim 1 or 2 wherein the fluorinating agent is selected from the group consisting of an N-fluoropiperidine, an N-fluoro pyridone, an N-fluoro sulphonamide, an N-fluoro pyridinium salt, an N-fluoro imide and an N-fluoro diazabicyclalkane.

4. A method as in claim 3 wherein the fluorinating agent is a 1-alkyl-4-fluoro-1,4-diazabicyclo[2,2,2]octane salt.

5. A method as in claim 4 wherein the fluorinating agent is a 1-chloromethyl-4-fluoro-1,4-diazabicyclo[2,2,2]octane salt.

6. A method as in claim 4 or 5 wherein the salt is a bis(tetrafluoroborate)salt.

7. A method as in any of claims 1 to 6 wherein the compound is contained in a solvent.

8. A method as in any of claims 1 to 7 wherein the reaction is carried out at a temperature in the range -60°C to +150°C.

9. A method as in claim 8 wherein the temperature is in the range 0°C to 100°C.

10. A method as in any preceding claim wherein the molar ratio of fluorinating agent to compound is in the range 0.5:1 to 10:1.

11. A method as in any preceding claim wherein the saturated carbon atom is a tertiary carbon atom.

12. A method as in any preceding claim wherein the saturated carbon atom forms part of a cyclic structure.

## Patentansprüche

1. Verfahren zum selektiven Fluorieren einer organischen Verbindung an einem nicht aktivierten, gesättigten sp³-hybridisierten Kohlenstoffatom, das den Schritt aufweist des Reagierens der Verbindung mit einem fluorierenden Mittel, das eine N-F-Bindung aufweist.

2. Verfahren nach Anspruch 1, wobei ein Wasserstoffatom an dem gesättigten Kohlenstoffatom durch ein Fluoratom ersetzt wird.

3. Verfahren nach Anspruch 1 oder 2, wobei das fluorierende Mittel ausgewählt ist aus der Gruppe bestehend aus einem N-Fluorpiperidin, einem N-Fluorpyridon, einem N-Fluorsulfonamid, einem N-Fluorpyridiniumsalz, einem N-Fluorimid und einem N-Fluordiazabicycloalkan.

4. Verfahren nach Anspruch 3, wobei das fluorierende Mittel ein 1-Alkyl-4-fluor-1,4-diazabicyclo[2.2.2]octansalz ist.

5. Verfahren nach Anspruch 4, wobei das fluorierende Mittel ein 1-Chlormethyl-4-fluor-1,4-diazabicyclo[2.2.2]octansalz ist.

6. Verfahren nach einem der Ansprüche 4 oder 5, wobei das Salz ein Bis(tetrafluorborat)salz ist.

7. Verfahren nach einem der Ansprüche 1 bis 6, wobei die Verbindung in einem Lösungsmittel enthalten ist.

8. Verfahren nach einem der Ansprüche 1 bis 7, wobei die Reaktion bei einer Temperatur im Bereich von -60°C bis +150°C durchgeführt wird.

9. Verfahren nach Anspruch 8, wobei die Temperatur im Bereich von 0°C bis 100°C liegt.

10. Verfahren nach einem der vorhergehenden Ansprüche, wobei das Molverhältnis des fluorierenden Mittels zu der Verbindung im Bereich von 0,5:1 bis 10:1 liegt.

11. Verfahren nach einem der vorhergehenden Ansprüche, wobei das gesättigte Kohlenstoffatom ein tertiäres Kohlenstoffatom ist.

12. Verfahren nach einem der vorhergehenden Ansprüche, wobei das gesättigte Kohlenstoffatom Teil einer cyclischen Struktur bildet.

## Revendications

1. Procédé pour la fluoration sélective d'un composé organique en un atome de carbone hybridé sp³ saturé non activé, le procédé comprenant la mise en réaction du composé avec un agent de fluoration contenant une liaison N-F.

2. Procédé selon la revendication 1, **caractérisé en ce qu'**un atome d'hydrogène, en l'atome de carbone saturé, est substitué par un atome de fluor.

3. Procédé selon l'une des revendications 1 ou 2, **caractérisé en ce que** l'agent de fluoration est choisi parmi le groupe consistant en N-fluoropipéridine, un N- fluoro pirydone, un N-fluoro sulphonamide, un sel N-fluoro pyridinium, un imide N-fluoro, et un N-fluoro diazabicyclalkane.

4. Procédé selon la revendication 3, **caractérisé en ce que** l'agent de fluoration est un sel 1-alkyl-4-fluoro-1,4-diazabicyclo[2,2,2] octane.

5. Procédé selon la revendication 4, **caractérisé en ce que** l'agent de fluoration est un sel 1-chlorométhyle-4-fluoro-1,4-diazabicyclo[2,2,2] octane.

6. Procédé selon la revendication 4 ou 5, **caractérisé en ce que** le sel est un sel bis(tétrafluoroborate).

7. Procédé selon l'une des revendications 1 à 6, **caractérisé en ce que** le composé est contenu dans un solvant.

8. Procédé selon l'une des revendications 1 à 7, **caractérisé en ce que** la réaction est réalisée à une température dans la gamme de -60°C à +150°C.

9. Procédé selon la revendication 8, **caractérisé en ce que** la température est dans la gamme de 0°C à 100°C.

10. Procédé selon l'une des revendications précédentes, **caractérisé en ce que** le ratio molaire d'agent de fluoration par rapport au composé est dans la gamme de 0,5 pour 1 jusqu'à 10 pour 1.

11. Procédé selon l'une des revendications précédentes, **caractérisé en ce que** l'atome de carbone saturé est un atome carbone tertiaire.

12. Procédé selon l'une des revendications précédentes, **caractérisé en ce que** l'atome de carbone saturé forme une partie d'une structure cyclique.
